# EUROPEAN PATENT SPECIFICATION

(11) **EP 1 700 556 B1**
(45) Date of publication and mention of the grant of the patent: **04.06.2014**
(21) Application number: 05356161.9
(22) Date of filing: 15.09.2005
(51) Int. Cl.: A47L 9/04

(54) **System and method for increasing inertia in a turbine brush**
System und Verfahren zur Erhöhung der Trägheit in einer Turbinenbürste
Système et procédé d'augmentation de l'inertie dans une brosse à turbine

(30) Priority: 10.03.2005 KR 2005019963; 15.04.2005 KR 2005031545
(43) Date of publication of application: 13.09.2006
(73) Proprietor: Samsung Gwangju Electronics Co., Ltd., Gwangju-city (KR)
(72) Inventor: Lee, Byung-jo, Buk-gu Gwangju-city (KR); Park, Yun-hee, c/o Samsung Gwangju Electronics, Gwangsan-gu Gwangju-city (KR); Yoo, Dong-hun, Gwangsan-gu Gwangju-city (KR); Choung, Myoung-sun, Buk-gu Gwangju-city (KR)
(74) Representative: Myon, Gérard Jean-Pierre

(56) References cited:
- EP-A- 0 780 085
- AT-B- 383 264
- DE-A1- 10 042 672
- DE-A1- 19 751 322
- DE-C1- 19 602 406
- GB-A- 489 776
- JP-A- 6 105 772
- US-A- 2 100 089

## Description

### BACKGROUND OF THE INVENTION

### Field of the invention

The present invention relates generally to the field of vacuum cleaners and the like. In particular embodiments, a turbine brush of a vacuum cleaner is rotated by a turbine to remove impurities on a surface being cleaned.

### Description of the Related Art

In general, vacuum cleaners comprise a brush member that contacts the surface being cleaned to draw in dust from the surface. Moving along the surface being cleaned, the brush member is typically rotated to scratch or beat the surface being cleaned, thereby separating the dust from the surface being cleaned. The separated dust is drawn into a main body of the vacuum cleaner by a suction force generated in the main body.

The brush member is typically rotated by a dedicated driving motor. The driving motor is mounted in connection with the brush member to selectively rotate the brush member. However, such connection between the driving motor and the brush member increases the complexity of the structure and increases manufacturing costs. Therefore, recently, a turbine unit has been introduced to rotate the brush member.

In structures that employ a turbine unit to rotate the brush member, the turbine unit is mounted on a suction path through which the dust is drawn in by the suction force generated in the main body. The turbine unit is rotated by air drawn in through the suction path, and the rotating force is supplied to the brush member through a belt. Accordingly, the brush member draws in dust while rotating in contact with the surface being cleaned.

However, when the turbine unit is used to rotate the brush member, the dust drawn in through the suction path may be caught in the turbine unit. When small particles such as hair and fine dust are caught in the turbine unit, the resulting friction may decrease the rotation of the turbine due to low inertia and low torque of the turbine unit.

Accordingly, the rotative force of the brush member connected with the turbine unit is affected, thereby reducing cleaning efficiency. AT-B-383 264 discloses a brush for a vacuum cleaner which comprises a brush body, a brush member mounted so as to oscillate relative to the brush body and a turbine rotated by air drawn in through a suction path of the brush body. The turbine is mechanically associated with a dynamo and the movement of the brush member is activated by an electromagnetic device which is electrically coupled with the dynamo. However, in AT-B-383 264, the brush member is not rotatably mounted to the brush body, it can only oscillate relative to the brush body.

JP-A-06 105772 and DE-C-196 02 406 each disclose a brush for a vacuum cleaner, comprising a brush member which is rotatably mounted to a brush body and driven by a turbine rotated by air drawn in through a suction path of the brush body. The turbine disclosed in JP-A-06 105772 has blades with a globally constant shape along their length, whereas the blades of the turbine disclosed in DE-C-196 02 406 extend only in the vicinity of the outermost circumference of the turbine, not from the rotational center of the turbine. Such turbine structures do not lead to an optimal rotative force of the brush member.

### SUMMARY OF THE INVENTION

It is to be understood that both the fallowing summary and the detailed description are exemplary and explanatory and are intended to provide further explanation of the invention as claimed. Neither the summary nor the description that follows is intended to define or limit the scope of the invention to the particular features mentioned in the summary or in the description.

In certain embodiments, the present invention may solve one or more of the above problems and/or disadvantages and may provide one or more of the advantages described herein.

In an exemplary embodiment, a turbine brush for a vacuum cleaner according to claim 1 is provided.

Particular embodiments are set out in the dependent claims.

### BRIEF DESCRIPTION OF THE DRAWING FIGURES

The accompanying drawings, which are incorporated herein and form a part of the specification, illustrate the present invention and, together with the description, further serve to explain the principles of the invention and to enable a person skilled in the pertinent art to make and use the invention.

FIG. 1 is a perspective view of a vacuum cleaner employing a turbine brush according to a first exemplary embodiment of the present invention;

FIG. 2 is an exploded, perspective view of the turbine brush of FIG. 1;

FIG. 3 is an exploded, perspective view of a driving unit of FIG. 2;

FIG. 4 is a sectional view of main elements in a state an inertia member of FIG. 3 is mounted in the driving unit;

FIG. 5 is a perspective view of a second embodiment of a turbine not forming part of the present invention;

FIG. 6 is a sectional view of the turbine taken on VI-VI line of FIG. 5;

FIG. 7 is a sectional view of a third embodiment of a turbine not forming part of the present invention; and

FIG. 8 is a sectional view of a fourth embodiment of a turbine not forming part of the present invention.

### DETAILED DESCRIPTION OF THE EXEMPLARY EMBODIMENTS

Hereinafter, exemplary embodiments of the present invention will be described in detail with reference to the accompanying drawing figures.

The present invention will now be explained in terms of exemplary embodiments. This specification discloses one or more embodiments that incorporate the features of this invention. The embodiment(s) described, and references in the specification to "one embodiment", "an embodiment", "an example embodiment", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, persons skilled in the art may effect such feature, structure, or characteristic in connection with other embodiments whether or not explicitly described.

In the following description, similar drawing reference numerals may be used for the same elements even in different drawings. The embodiments described, and their detailed construction and elements, are merely provided to assist in a comprehensive understanding of the invention. Thus, it is apparent that the present invention can be carried out in a variety of ways, and does not require any of the specific features described herein. Also, well-known functions or constructions are not described in detail since they would obscure the invention with unnecessary detail.

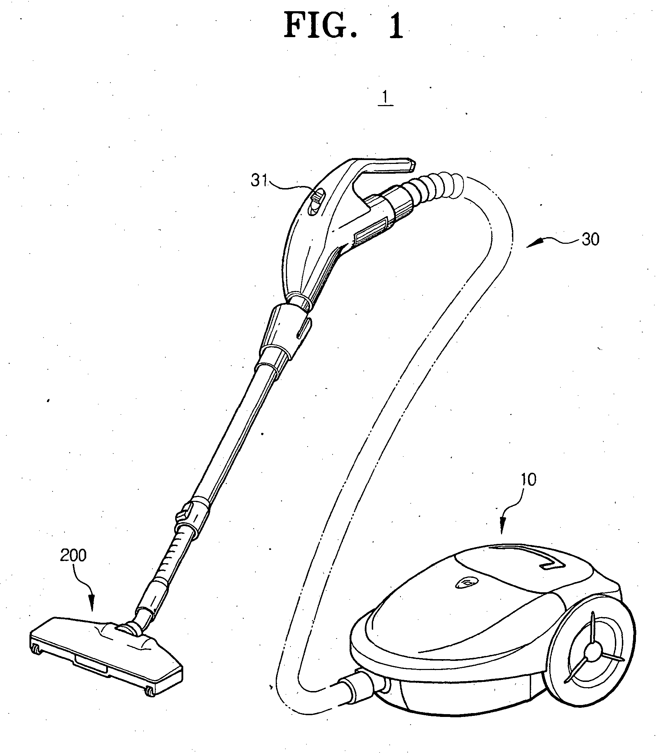

FIG. 1 is a perspective view of a vacuum cleaner employing a turbine brush according to the first embodiment of the present invention.

Referring to FIG. 1, in an exemplary embodiment the vacuum cleaner 1 comprises a cleaner body 10 including a vacuum generator (not shown) and a dust collecting chamber (not shown), a turbine brush 200 for drawing in dust from a surface being cleaned, and a connection member 30 for connecting the cleaner body 10 and the turbine brush 200. The connection member 30 comprises an operation switch 31 for turning on and off the vacuum cleaner 1.

The vacuum generator (not shown) generates a suction force for drawing in the dust separated from the surface being cleaned. General driving motors can be applied for the vacuum generator. The dust collecting chamber (not shown) collects therein the dust drawn in by the suction force of the vacuum generator.

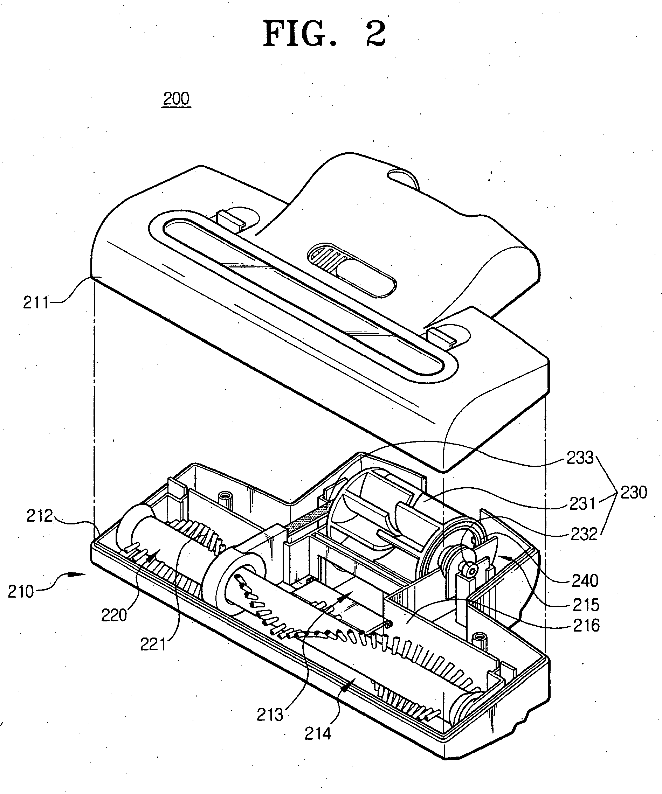

As shown in the exemplary embodiment of FIG. 2, the turbine brush 200 comprises a turbine brush body 210, a brush member 220, a driving unit 230 and an inertia member 240.

In this example, the turbine brush body 210 is connected to the cleaner body 10 through the connection member 30 to be transmitted with the suction force from the vacuum generator. The turbine brush body 210 comprises upper and lower frames 211 and 212, a suction path 213, a brush member receiving portion 214, and a driving unit receiving portion 215.

The upper and the lower frames 211 and 212 are connected to face each other, thereby constituting an exterior of the turbine brush body 210. The lower frame 212 has a suction opening (not shown) for drawing in external air from the surface being cleaned.

The suction path 213 is formed for the dust drawn in from the surface being cleaned to move to the connection member 30. For this, the suction path 213 provides fluid connection between the brush member receiving portion 214 and the driving unit receiving portion 215, such that the drawn-in dust is guided into the dust collecting chamber of the cleaner body 10 sequentially passing through the brush member 220, the driving unit 230 and the connection member 30.

More specifically, the brush member receiving portion 214 is fluidly connected to the suction opening that draws in the external air by the suction force. The driving unit receiving portion 215 is fluidly connected to the connection member 30, such that the dust drawn in through the suction opening and moved along the brush member 220 and the suction path 213 is guided to the connection member 30. The brush member receiving portion 214 and the driving unit receiving portion 215 are sectioned by a partition 216 formed at the lower frame 212.

The brush member 220 is rotatably mounted to the turbine brush body 210. To this end, the brush member 220 is received in the brush member receiving portion 214 and rotatably supported by the turbine brush body 210 with both ends thereof. A plurality of bristles 221 are implanted at certain intervals along an outer circumference of the brush member 220, and the bristles 221 are exposed to the outside through the suction opening.

Rotating together with the brush member 220, the bristles 221 scratch or beat the dust on the surface being cleaned, thereby separating the dust from the surface being cleaned. The separated dust is drawn in through by the suction force generated in the cleaner body 10 and guided into the dust collecting chamber.

Although the bristles 221 of this embodiment are illustrated in a spiral form symmetrically implanted with respect to a center portion of the outer circumference of the brush member 220, the present invention is not limited so. For example, the bristles 221 may be formed parallel with an axial direction of the brush member 220 at certain intervals. That is, the bristles 221 can be formed in any various types capable of separating the dust from the surface being cleaned.

The driving unit 230 is rotatably mounted to the driving unit receiving portion 215 which is provided on the suction path 213 in the turbine brush body 210, so as to drive the brush member 220. For this, the driving unit 230 comprises a turbine 231, a turbine shaft 232 and a power transmitter 233.

The turbine 231 is rotated by the air which is drawn in through the suction path 213 by the suction force generated from the vacuum generator. The turbine 231 comprises a plurality of blades 234 inwardly protruded from opposite ends of the turbine 231. More preferably, the blades 234 formed from the opposite ends are alternately disposed, such that the turbine 231 1 can be rapidly rotated by the air drawn in along the suction path 213.

The turbine shaft 232 is inserted in a rotational center of the turbine 231 and supported by the lower frame 212 with opposite ends thereof, such that the turbine 231 is rotatably supported by the turbine brush body 210.

The power transmitter 233 conveys a rotative force of the turbine 231 to the brush member 220. Preferably, a timing belt connecting the brush member 220 and the turbine shaft 232 of the turbine 231 is used for the power transmitter 233.

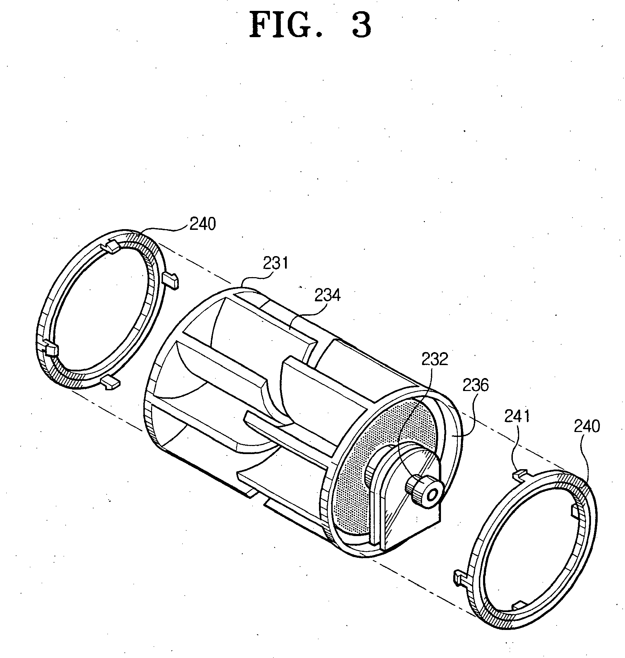

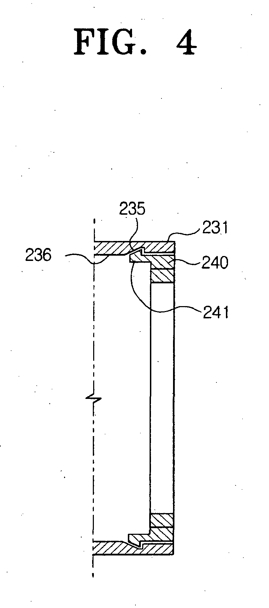

As shown in FIGS. 3 and 4, the inertia member 240 is mounted to the turbine 231 for the driving unit 230 to add inertia to a driving force for operating the brush member 220.

In other words, the inertia member 240 is implemented by a mass which rotates together with the turbine 231 for increasing the inertia by adding mass to the rotative force of the turbine 231. To this end, the inertia member 240 comprises a hook 241 for engagement with a hook hole 235 formed on an inner circumference 236 of the turbine 231.

More preferably, the inertia member 240 is formed as a ring in a corresponding shape to the inner circumference 236 of the turbine 231 and fixed in tight contact with opposite sides of the inner circumference 236 of the turbine 231 by the hook 241.

In the present embodiment, the inertia member 240 is fixed to the inner circumference 236 of the turbine 231 by the hook 241; however, the present invention is not limited to this structure. The inertia member 240 may be attached by a dedicated fixing means such as adhesive or integrally formed with the turbine 231.

Hereinbelow, operational relationship between the vacuum cleaner 1 and the turbine brush 200 will be described with reference to FIGS. 1 through 4.

As shown in FIG. 1, as the operation switch 31 of the connection member 30 is turned on, with the turbine brush 200 disposed to face the surface being cleaned, a suction force is generated from a driving motor (not shown) mounted in the cleaner body 10. The external air is drawn into the turbine brush body 210 by the suction force, and accordingly, the driving unit 230 is operated.

The turbine 231 of the driving unit 230 is rotated together with the inertia member 240, and the rotative force of the turbine 231 is transmitted to the brush member 220 through the power transmitter 233. Therefore, the brush member 220 is rotated to scratch and beat the dust on the surface being cleaned, thereby separating the dust from the surface being cleaned.

The dust separated by the brush member 220 is drawn in by the suction force and, as passing through the suction path 213 and the connection member 30, collected in the dust collecting chamber in the cleaner body 10.

The turbine 231 is rotated with the inertia member 240 so that the inertia can be added to the turbine 231 and increase the rotative force.

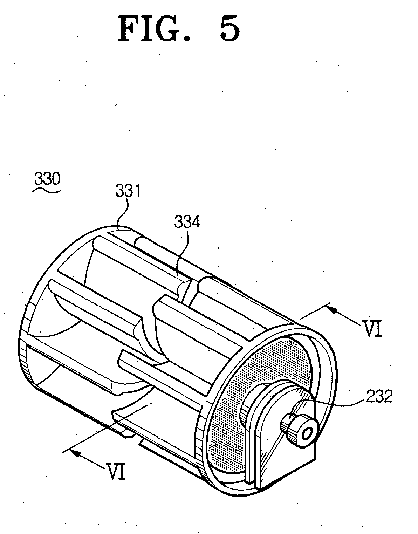

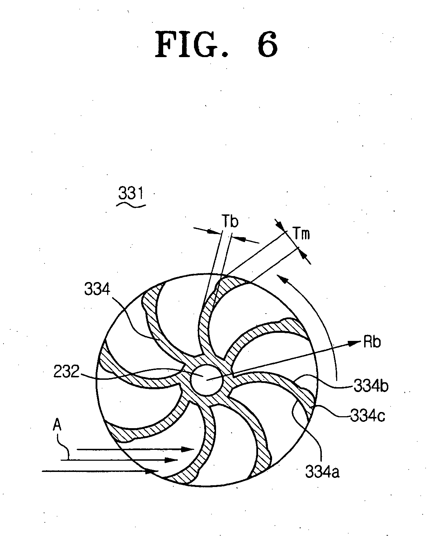

The turbine brush according to the second embodiment not forming part of the present invention comprises the turbine brush body 210 and the brush member 220 and may have, for example, the same structures as shown in FIG. 2 and the driving unit 330 with the same structures as shown in FIGS. 5 and 6.

The detailed descriptions and drawings of same structures as the aforementioned first embodiment will be omitted in the present second embodiment, and the following third and the fourth embodiments not forming part of the present invention.

The driving unit 330 according to the second embodiment not forming part of the present invention comprises a second embodiment of a turbine 331, the turbine shaft 232, and the power transmitter 233 (refer to FIG. 2). The technical constructions of the turbine shaft 232 and the power transmitter 233 may be, for example, the same as described with reference to FIG. 2.

The turbine 331 is rotated by air drawn in through the suction path 213 and having a plurality of blades 334 with thickness-varying portions in a direction of radius *Rb.*

More specifically, arcs of the blades 334 of the turbine 331 are uneven in a spiral direction on opposite surfaces 334b to surfaces 334a encountering resistance of air A drawn in by a suction force.

The thickness *Tm* of a distal end 334c of the blade 334 may be greater than the other portion of the blade 334 due to the uneven arc. The distal end 334c of the blade 334 indicates the distal end 334c furthest from the turbine shaft 232 in the radius direction *Rb.*

The arc of the blade 334 is uneven at the distal end 334c, and therefore, the thickness *Tm* of the distal end 334c is greater than the thickness *Tb* of the blade 334. The distal end 334c of the blade 334 may be made of the same material as the blade 334 or may be made of material different from the blade 334.

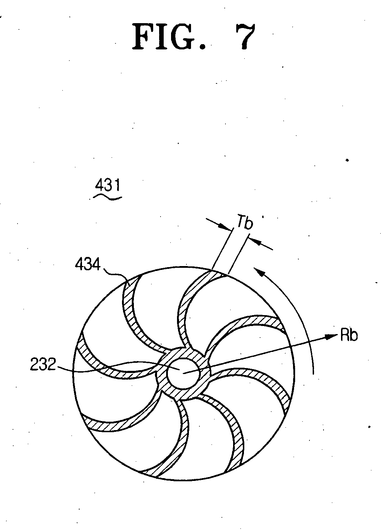

Referring to FIG. 7, the turbine brush according to the third embodiment not forming part of the present invention is characterized of a third embodiment of a turbine 431 having a plurality of blades 434 with increasing thickness *Tb* in the direction further away from the turbine shaft 232. The thickness *Tb* of the blade 434 increases in proportion to the length in the radius direction *Rb.* Therefore, more weight can be added to the blade 434 and increase the inertia.

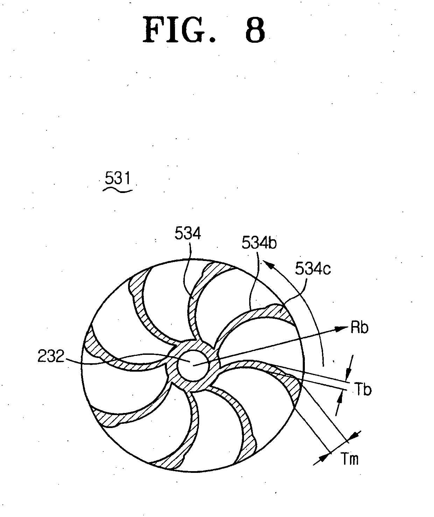

Referring to FIG. 8, the turbine brush according to the fourth embodiment not forming part of the present invention is characterized of a fourth embodiment of a turbine 531 having a plurality of blades 534 with increasing thickness *Tb* in a direction of the radius *Rb* and arcs of the blades 534 being uneven in a spiral direction on opposite surfaces 534b of the blades 534. The uneven arcs may preferably be formed at the distal ends 534c of the blades 534.

Therefore, all the distinguishing features of blades 334 and 434 according to the second and the third embodiments are applied to the blades 534 according to the present embodiment. Therefore, the blades 534 can effectively increase the rotative force.

According to the above structure, although dust, hair, and other small items are drawn in, owing to the inertia member 240 or blades 334, 434, 534 with varying thickness *Tb* in the radius direction *Rb,* inertia can be increased and therefore, the effect of these particles on the rotative force of the turbine 331, 431, 531 is minimized.

If the turbine brush 200 is applied according to the embodiments, the turbine 231, 331, 431 or 531 driving the brush member 220 rotates together with the inertia member 240, and may not forming part of the invention have blades 334, 434 or 534 with a thickness-varying portion in a direction of the radius *Rb,* such that a torque, that is the rotative force according to the centrifugal force of the turbine 231, 331, 431 or 531, can increase.

The effect of small particles such as fine dust and hair on the turbine is reduced by the inertia added to the turbine, thereby improving cleaning effciency.

While the invention has been shown and described with reference to certain embodiments thereof, it will be understood by those skilled in the art that various changes in form and details may be made therein without departing from the scope of the invention as defined by the appended claims.

## Claims

1. A turbine brush (200) for a vacuum cleaner (1), comprising:
a turbine brush body (210) adapted to be connected to a cleaner body (10) in which a suction force is generated, and having a suction path (213) therein;
a brush member (220) rotatably mounted to the turbine brush body;
a driving unit (230, 330) rotatably mounted in the turbine brush body to drive the brush member and comprising a turbine (231, 331,431,531) rotated by air drawn in through the suction path (213) and having a plurality of blades (234, 334, 434, 534), a turbine shaft (232) disposed at a rotational center of the turbine, and a power transmitter (233) for conveying a rotative force of the turbine to the brush member (220); **characterized in that** it comprises
two inertia members (240) detachably mounted to the turbine (231, 331, 431, 531) and rotated together with the turbine to add inertia to a driving force of the driving unit,
wherein the two inertia members (240) comprise a hook (241) for engagement with a hook hole (235) formed on opposite ends of the turbine (231, 331, 431, 531), have an annular shape and are fixed in tight contact with opposite sides of the inner circumference of the turbine (231, 331, 431, 531).

2. The turbine brush of claim 1, wherein at least one blade (334, 434, 534) of the plurality of blades has a greater thickness at a distal end (334c, 534c) thereof than at a proximate end thereof.

3. The turbine brush of claim 1, wherein the thickness (Tb) of at least one blade (434,534) of the plurality of blades increases with distance from the rotational center.

4. The turbine brush of claim 3, wherein the blade (534) has an uneven arc at a distal end (534c) on an opposite surface (534b) of a surface encountering a resistance of the intake air.

5. The turbine brush of claim 1, wherein the power transmitter (233) includes a timing belt connecting the turbine shaft (232) and the brush member (220) to transmit power.

6. The turbine brush of claim 1, wherein the driving unit (230, 330) is disposed in the suction path (213) in the turbine brush body (210).

## Patentansprüche

1. Eine Turbinenbürste (200) für einen Staubsauger (1), aufweisend:
einen Turbinenbürstenkörper (210), der angepasst ist, mit einem Staubsaugerkörper (10), in dem eine Saugkraft erzeugt wird, verbunden zu sein, und der einen Ansaugpfad (213) darin aufweist,
ein Bürstenelement (220), das drehbar an dem Turbinenbürstenkörper montiert ist,
eine Antriebseinheit (230, 330), die drehbar in dem Turbinenbürstenkörper montiert ist, um das Bürstenelement anzutreiben, und die aufweist: eine Turbine (231, 331, 431, 531), die durch Luft rotiert wird, die durch den Ansaugpfad (213) hindurch angesaugt wird, und die eine Mehrzahl von Schaufeln (234, 334, 434, 534) aufweist, eine Turbinenwelle (232), die in einem Rotationszentrum der Turbine angeordnet ist, und eine Energieübertragungseinrichtung (233) zum Übertragen einer Rotationskraft der Turbine auf das Bürstenelement (220),
**dadurch gekennzeichnet, dass** sie aufweist:
zwei Trägheitselemente (240), die lösbar an der Turbine (231, 331, 431, 531) montiert sind und zusammen mit der Turbine rotiert werden, um einer Antriebskraft der Antriebseinheit Trägheit hinzuzufügen,
wobei die beiden Trägheitselemente (240) einen Haken (241) für einen Eingriff mit einem an entgegengesetzten Enden der Turbine (231, 331, 431, 531) ausgebildeten Hakenloch (235) aufweisen, eine ringförmige Form haben und in engem Kontakt mit gegenüberliegenden Seiten des Innenumfangs der Turbine (231, 331, 431, 531) befestigt sind.

2. Die Turbinenbürste gemäß Anspruch 1, wobei mindestens eine Schaufel (334, 434, 534) der Mehrzahl von Schaufeln an einem distalen Ende (334c, 534c) davon eine größere Dicke als an einem benachbarten Ende davon hat.

3. Die Turbinenbürste gemäß Anspruch 1, wobei die Dicke (Tb) von mindestens einer Schaufel (434, 534) der Mehrzahl von Schaufeln mit Abstand von dem Rotationszentrum größer wird.

4. Die Turbinenbürste gemäß Anspruch 3, wobei die Schaufel (534) einen ungleichmäßigen Bogen an einem distalen Ende (534c) an einer entgegengesetzten Fläche (534b) einer Fläche hat, die auf einen Widerstand der Ansaugluft trifft.

5. Die Turbinenbürste gemäß Anspruch 1, wobei die Energieübertragungseinrichtung (233) einen Antriebsriemen aufweist, der die Turbinenwelle (232) und das Bürstenelement (220) miteinander verbindet, um Energie zu übertragen.

6. Die Turbinenbürste gemäß Anspruch 1, wobei die Antriebseinheit (230, 330) in dem Ansaugpfad (213) in dem Turbinenbürstenkörper (210) angeordnet ist.

## Revendications

1. Brosse à turbine (200) pour un aspirateur (1), comprenant :
un corps de brosse à turbine (210) apte à être relié à un corps d'aspirateur (10) dans lequel une force d'aspiration est générée, et comportant une voie d'aspiration (213) dans celui-ci ;
un organe de brosse (220) monté de manière à pouvoir tourner sur le corps de brosse à turbine ;
une unité d'entraînement (230, 330) montée de manière à pouvoir tourner dans le corps de brosse à turbine pour entraîner l'organe de brosse et comprenant une turbine (231, 331, 431, 531) tournée par l'air aspiré à travers la voie d'aspiration (213) et comportant une pluralité de pales (234, 334, 434, 534), un arbre de turbine (232) disposé à un centre de rotation de la turbine, et un transmetteur de puissance (233) pour transmettre une force de rotation de la turbine à l'organe de brosse (220) ; **caractérisée en ce qu'**elle comprend :
deux organes d'inertie (240) montés de manière détachable sur la turbine (231, 331, 431, 531) et tournés avec la turbine pour ajouter de l'inertie à une force d'entraînement de l'unité d'entraînement,
dans laquelle les deux organes d'inertie (240) comprennent un crochet (241) pour se mettre en prise avec un trou de crochet (235) constitué à des extrémités opposées de la turbine (231, 331, 431, 531), ils ont une forme annulaire et ils sont fixés en contact étroit avec des côtés opposés de la circonférence intérieure de la turbine (231, 331, 431, 531).

2. Brosse à turbine selon la revendication 1, dans laquelle au moins une pale (334, 434, 534) de la pluralité de pales a une épaisseur à une extrémité distale (334c, 534c) de celle-ci qui est supérieure à celle à une extrémité proximale de celle-ci.

3. Brosse à turbine selon la revendication 1, dans laquelle l'épaisse (Tb) d'au moins une pale (434, 534) de la pluralité de pales augmente avec une distance par rapport au centre de rotation.

4. Brosse à turbine selon la revendication 3, dans laquelle la pale (534) présente un arc inégal à une extrémité distale (534c) sur une surface opposée (534b) d'une surface rencontrant une résistance de l'air d'admission.

5. Brosse à turbine selon la revendication 1, dans laquelle le transmetteur de puissance (233) comprend une courroie de distribution reliant l'arbre de turbine (232) et l'organe de brosse (220) pour transmettre une puissance.

6. Brosse à turbine selon la revendication 1, dans laquelle l'unité d'entraînement (230, 330) est disposée dans la voie d'aspiration (213) dans le corps de brosse à turbine (210).
